Europäisches Patentamt

European Patent Office

Office européen des brevets

(19)

(11) Numéro de publication: **0 075 523
B1**

(12)

# FASCICULE DE BREVET EUROPEEN

(45) Date de publication du fascicule du brevet:
**27.12.85**

(51) Int. Cl.⁴: **A 61 K 35/80**

(21) Numéro de dépôt: **82401721.4**

(22) Date de dépôt: **23.09.82**

(54) Nouveaux médicaments à base d'extraits d'algues, et formulations correspondantes.

(30) Priorité: **23.09.81 FR 8117942**

(43) Date de publication de la demande:
**30.03.83 Bulletin 83/13**

(45) Mention de la délivrance du brevet:
**27.12.85 Bulletin 85/52**

(84) Etats contractants désignés:
**AT BE CH DE GB IT LI LU NL SE**

(56) Documents cités:
**FR - M - 5 576**
**GB - A - 1 141 573**
**GB - A - 1 141 821**
**US - A - 4 162 308**
**US - A - 4 162 309**

**BIOLOGICAL ABSTRACTS, vol. 68, no. 3, 1979, no. 17441, Philadelphia- Pennsylvania (USA); N.Z.I.S. SHIL'TSOV et al.: "Marine organisms as a source of biological active substances"**

(73) Titulaire: **LABORATOIRES GOEMAR S.A., Avenue du Général Patton Boîte Postale 55, F-35403 Saint-Malo (FR)**

(72) Inventeur: **Herve, René, Les Tertres Route de Lamballe Pluduno, F-22130 Plancoet (FR)**
Inventeur: **Percehais, Serge, 8, rue d'Orleans, F-35400 Saint Malo (FR)**

(74) Mandataire: **Ranguis, Patrick Frédéric et al, Cabinet Beau de Loménie 55, rue d'Amsterdam, F-75008 Paris (FR)**

## Description

La présente invention concerne l'utilisation en thérapeutique d'extraits de certaines algues préparés d'une manière spéciale.

On sait qu'il existe plusieurs familles d'algues.

Les algues qui sont utilisées selon la présente invention appartiennent à la famille des algues brunes ou phéophycées.

L'invention vise particulièrement la découverte de propriétés surprenantes des algues brunes suivantes:

— Bifurcaria Rotunda
— Fucus Vesiculosus
— Ascophyllum Nodosum
— Pelvetia Canaliculata

Dans le brevet FR-M-5 576 on a déjà proposé une pondre d'algue brune dont les particules mesurent de 4 à 5 μm, pour des fins thérapeutiques. Cette pondre est néammoins obtenée par un procédé détunisant certains agents actifs.

Les propriétés découvertes selon l'invention sont étroitement liées au traitement spécial de ces algues qui conduit à un produit présentant des caractéristiques particulières physicochimiques.

Les algues sont traitées de la minière suivante.

On récolte les algues, et on les lave en piscine afin de les débarrasser des animalcules et du sable.

On leur fait subir une surgélation, notamment dans un surgélateur à plaques, à —10/—30°C, afin de permettre la conservation des éléments utiles.

On effectue ensuite en général une conservation en chambre froide en raison du fait que les récoltes sont saisonnières.

Les algues sont ensuite soumises à un cryobroyage (on peut utiliser deux broyeurs en cascade sous azote liquide) puis à un laminage (on peut utiliser une machine à cylindres) et enfin à une homogénéisation. On obtient ainsi une »bouillie-mère« dont les particules constitutives présentent une dimension de 6 à 20 μm environ. Cette bouillie-mère est aussi appelée »crème d'algues«.

On fait passer cette bouillie-mère sur une décanteuse à grande vitesse qui donne deux produits, d'une part le gâteau que l'on dénomme »base d'algues« et d'autre part le jus de décantation que l'on dénomme »protoexoplasma d'algues«.

Alternativement, à titre indicatif, cette bouillie-mère peut être également conditionnée en l'état.

Egalement à titre indicatif, il est possible d'effectuer une lyophilisation aussi bien de la crème d'algues que du protoexoplasma d'algues pour donner une forme sèche qui peut donner lieu par exemple à la fabrication de comprimés.

Cette suite d'opérations est bien entendu valable pour chaque variété d'algues.

Dans le domaine de la préparation de ces différents extraits, on pourra se référer utilement au brevet français FR-A-2 287 943 déposé le 18 octobre 1974 au nom de HERVE et ROULLIER.

Le gâteau d'algues permet de fabriquer par exemple des savons et analogues, car il contient environ 4 à 6% de cellulose 38 à 40% d'alginates insolubles, ainsi que des vitamines liposolubles.

Cependant, la présente invention concerne des médicaments utiles pour augmenter le taux des $\gamma$-globulines en mèdecine humaine ou vétérinaire, et à action anti-virsle, caractérisés en ce qu'ils consistent en »protoexoplasma« d'elgues brunes qui est le jus de décantation, obtenu lors du passage sur décanteuse à grande vitesse de la bouillie obtenue par cryobroyage des algues brunes.

Les essais effectués on mis en évidence pour ces produits des propriétés d'augmentation du taux des anti-corps.

On a noté en particulier une augmentation du taux de $\gamma$-globulines, principalement au niveau des immuno-G.

Les essais ont été effectués par voie orale (sirops, ampoules buvables).

Ainsi, les produits selon l'invention sont utiles pour traiter tous les états pathologiques dans le traitement desquels une augmentation ou un maintien du pouvoir de défense de l'organisme est nécessaire, et provoquent une immunothérapie à long terme. Des essais effectués sur des rats de souche Witstar ont ainsi mis en évidence les propriétés suivantes:

— augmentation nette ou modérée des immuno-globulines G (le taux des immuno-globulines A des immonu-globulines M n'est pas modifié et reste donc normal; ceci est également valable pour les taux d'albumine, orosomucoïde, alpha 1 antitrypsine, haptoglobine, gamma 2 macroglobuline, lipoprotéines, transferrine, hemopexine, et béta 1 A globuline.

Sans vouloir être limitée d'une quelconque façon, la Demanderesse pense que ce phénomène est dû à l'existence d'une corrélation entre l'augmentation du zinc plasmatique et l'augmentation des immuno-globulines G.

— non augmentation de l'ACTH (acide homone-adrénocorticotropique) ce qui semble introduire une activité au niveau des surrénales.
— pas d'augmentation du taux de FSH (hormone de croissance).
— NFS normales. (numération formule sanguine).
— Rapport albumine/globuline diminué.
— Orientation du métabolisme dans le sens de l'anabolisme (dosage de métabolites aminés du type méthylhistidine dans l'urine).
— L'hormone adrénocorticotropique est peu secrétée sous l'influence de stimulis extérieurs (action prévisible au niveau du rétrocontrôle négatif hormonal).

— Le même phénomène semble s'exercer au niveau de l'hormone somatotrope.

Des essais ont été effectués chez le rat comme il a été dit ci-dessus. On a administré 0,02 ml de protoexoplasma d'Ascophyllum Nodosum par jour et par 100 g de poids corporel, par voie orale. On a noté une augmentation de 10 à 20% du taux de $\gamma$-globulines, ainsi qu'une augmentation du poids des thymus de 4 à 30% (4 à 10% chez la femelle et 10 à 30% chez le mâle) ainsi qu'un doublement du taux de vitamine A dans le foie des animaux traités.

Un essai a également été effectué sur un patient auquel on a administré chaque jour pendant un mois, 5 à 10 ml du même protoexoplasma, matin et soir. Au bout d'un mois on a noté une augmentation de 40% du taux de $\gamma$-globulines.

On a de plus noté que le rapport albumine/globuline diminue d'environ 20% au cours de cette expérimentation (1,72 → 1,38).

### Activite antivirale a'ascophyllum nodosum

#### Sur l'herpes Type I

Titrage par la méthode des plages.

Cette technique permet de mesurer très précisément la réduction, par un inhibiteur donné, de la virulence d'un virus.

#### Le principe en est le suivant

Des flacons de culture contenant une couche continue de cellules sensibles sont inoculés par une série de dilutions de 10 en 10 du virus à étudier. Après une heure d'absorption, la culture est recouverte de gélose. Le virus se multiplie alors dans les cellules où il a pénétré mais il ne peut pas disséminer et aller infecter des cellules lointaines. On n'observera donc pas de destruction du tapis cellulaire mais seulement des lésions locales (plages), chaque lésion étant due à une particule virale.

Une série de boîtes témoins sert à déterminer le titre du virus qui est exprimé en unités formant plage par ml (U F P/ml).

Dans la série essai, on ajoute l'extrait à étudier dans le milieu de culture avant inoculation du virus et dans la gélose.

#### Conditions expérimentales

Cet essai est réalisé avec un extrait alcoolique d'Ascophyllum préparé au laboratoire à partir du broyat d'algues fraîches; extrait qui est évaporé à sec, repris par l'eau distillée puis stérilisé par filtration sur filtre Millipore®.

Le filtrat dont 1 ml correspond à 1 g d'algue fraîche, est dilué au 1/20, 1/40, 1/200 pour la mesure de la réduction du titre viral.

#### Résultats

| | Titre viral U F P/ml | Observations |
|---|---|---|
| Témoin | $15 \times 10^6$ | |
| Essai 1/20 | 0 | Toxicité limitée |
| Essai 1/40 | 0 | Réduction du titre de 100% |
| Essai 1/200 | $15 \times 10^6$ | Inactif |

#### Conclusions

A la dilution 1/40, Ascophyllum Nodosum est donc très efficace puisque la multiplication de l'Herpès est complètement inhibée.

Les nouveaux médicaments selon l'invention seront donc utiles par voie orale à la dose indiquée ci-dessus pour lutter en médecine humaine contre des maladies telles que l'herpès et toute affection à virus dont la structure nucléique est équivalente.

Ces nouveaux médicaments seront également utiles en médecine vétérinaire et permettront par exemple une réduction de la quantité d'antibiotiques nécessaires pour protéger les animaux, grâce à une protection accrue de l'animal due à l'augmentation du taux de $\gamma$-globulines.

### Revendications pour les états contractants BE, CH, DE, GB, IT, LI, LU, NL, SE

1. Médicaments utiles pour augmenter le taux des $\gamma$-globulines en médecine humaine ou vétérinaire, et à action anti-virale, caractérisés en ce qu'ils consistent en »protoexoplasma« d'algues brunes qui est le jus de décantation obtenu lors du passage sur décanteuse à grande vitesse de la bouillie obtenue par cryobroyage des algues brunes.

2. Médicaments selon la revendication 1, caractérisés en ce que la dimension des particules du protoexoplasma est d'environ 6 à 20 µm.

3. Médicaments selon la revendication 1 ou 2, caractérisés en ce que les algues brunes utilisées sont choisies parmi les suivantes:

— Bifurcaria Rotunda
— Fucus Vesiculosus
— Ascophyllum Nodosum
— Pelvetia Canaliculata

4. Médicaments selon la revendication 1 à 3, caractérisés en ce que l'algue brune utilisée est la Ascophyllum Nodosum.

5. Forme pharmaceutique d'administration par voie arole des médicaments selon l'une quelconque des revendications 1 à 4 sous forme orale unitaire de 5 à 10 ml.

## Revendications pour l'état contractant AT

1. Utilisation de »protoexoplasma« d'algues brunes, qui est le jus de décantation obtenu lors du passage sur décanteuse à grande vitesse de la bouillie obtenue par cryobroyage des algues brunes, pour la préparation de médicaments utiles pour augmenter le taux des gamma-globulines en médicine humaine ou vétérinaire, et à action anti-virale.

2. Utilisation selon la revendication 1, caractérisée en ce que la dimension des particules du protoexoplasma est d'environ 6 à 20 $\mu$m.

3. Utilisation selon la revendication 1 ou 2, caractérisée en ce que les algues brunes utilisées sont choisies parmi les suivantes:

— Bifurcaria Rotunda
— Fucus Vesiculosus
— Ascophyllum Nodosum
— Pelvetia Canaliculata

4. Utilisation selon la revendication 1 à 3, caractérisée en ce que l'algue brune utilisée est la Ascophyllum Nodosum.

5. Utilisation selon une forme pharmaceutique d'administration par voie orale unitaire de 5 à 10 ml d'un médicament obtenu selon l'une des revendications 1 à 4.

## Patentansprüche für die Vertragsstaaten BE, CH, DE, GB, IT, LI, LU, NL, SE

1. Medikamente, die zur Erhöhung des $\gamma$-Globulinspiegels in der Human- oder Veterinärmedizin nützlich sind und antivirale Wirkung zeigen, dadurch gekennzeichnet, daß sie aus »Protoexoplasma« von Braunalgen bestehen, d. h. der Dekantierungsflüssigkeit, die beim Hochgeschwindigkeitszentrifugieren der durch Kryozerkleinerung von Braunalgen entstandenen Brühe erhalten wird.

2. Medikamente nach Anspruch 1, dadurch gekennzeichnet, daß die Dimension der Protoexoplasmateilchen etwa 6 bis 20 $\mu$m beträgt.

3. Medikamente nach Anspruch 1 oder 2, dadurch gekennzeichnet, daß die verwendeten Braunalgen ausgewählt sind aus den folgenden:

— Bifurcaria Rotunda
— Fucus Vesiculosus
— Ascophyllum Nodosum
— Pelvetia Canaliculata.

4. Medikamente nach Anspruch 1 bis 3, dadurch gekennzeichnet, daß die verwendete Braunalge Ascophyllum Nodosum ist.

5. Pharmazeutische Verabreichungsform auf oralem Weg der Medikamente nach einem der Ansprüche 1 bis 4 in oraler Einheitsform von 5 bis 10 ml.

## Patentansprüche für den Vertragsstaat AT

1. Verwendung von »Protoexoplasma« von Braunalgen, d. h. der Dekantierungsflüssigkeit, die beim Hochgeschwindigkeitszentrifugieren der durch Kryozerkleinerung von Braunalgen entstandenen Brühe erhalten wird, zur Herstellung von Medikamenten, die zur Erhöhung des $\gamma$-Globulinspiegels in der Human- oder Veterinärmedizin nützlich sind und antivirale Wirkung zeigen.

2. Verwendung nach Anspruch 1, dadurch gekennzeichnet, daß die Dimension der Protoexoplasmateilchen etwa 6 bis 20 $\mu$m beträgt.

3. Verwendung nach Anspruch 1 oder 2, dadurch gekennzeichnet, daß die verwendeten Braunalgen ausgewählt sind aus den folgenden:

— Bifurcaria Rotunda
— Fucus Vesiculosus
— Ascophyllum Nodosum
— Pelvetia Canaliculata.

4. Verwendung nach Anspruch 1 bis 3, dadurch gekennzeichnet, daß die verwendete Braunalge Ascophyllum-Nodosum ist.

5. Verwendung gemäß einer pharmazeutischen Verabreichungseinheitsform auf oralem Weg von 5 bis 10 ml eines nach einem der Ansprüche 1 bis 4 erhaltenen Medikamentes.

## Claims for the contracting states BE, CH, DE, GB, IT, LI, LU, NL, SE

1. Use of »protoexoplasma« of brown algae which is the decanting liquor resulting from the passage at high speed over a decanter of the pulp obtained by cryogrinding of the brown algae, for the preparation of drugs useful for raising the rate of gamma globulins in human or veterinary medecine, and with an anti-virus action.

2. Use according to claim 1, characterized in that the dimension of the protoexoplasma particles is about 6 to 20 $\mu$m.

3. Use according to claim 1 or 2, characterized in that the brown algae used are selected from the followings:

— Bifurcaria Rotunda
— Fucus Vesiculosus
— Ascophyllum Nodosum
— Pelvetia Canaliculata

4. Use according to claim 1 to 3, characterized in that the brown algae used is Ascophyllum Nodosum.

5. Use according to a unit pharmaceutical form of administration by oral route of 5 to 10 ml of a drug obtained according to anyone of claims 1 to 4.

## Claims for the contracting state AT

1. Drugs useful for raising the rate of gamma globulins in human or veterinary medecine, and with anti-virus action, characterized in that they consist in protoexoplasma of brown algae which is the decanting liquor resulting from the passage at high speed over a decanter of the pulp obtained by cryogrinding of the brown algae.

2. Drugs according to claim 1, characterized in that the dimension of the protoexoplasma particles is about 6 to 20 μm.

3. Drugs according to claim 1 or 2, characterized in that the brown algae used are selected from the followings:

— Bifurcaria Rotunda
— Fucus Vesiculosus
— Ascophyllum Nodosum
— Pelvetia Canalicuta

4. Drugs according to claim 1 to 3, characterized in that the brown algae used is Ascophyllum Nodosum.

5. Pharmaceutical form of administration by oral route of the drugs according to anyone of claims 1 to 4 in unit oral form of 5 to 10 ml.